# EUROPEAN PATENT APPLICATION

(11) **EP 1 637 523 A1**
(43) Date of publication of application: **22.03.2006**
(21) Application number: 04746142.1
(22) Date of filing: 18.06.2004
(51) Int. Cl.: C07D 239/90, C07D 239/91, C07D 495/04, C07D 487/04

(54) **PROCESS FOR PRODUCING PYRIMIDIN-4-ONE COMPOUND**

(30) Priority: 18.06.2003 JP 2003172873; 24.06.2003 JP 2003179077; 16.07.2003 JP 2003197904; 11.08.2003 JP 2003291426; 01.10.2003 JP 2003342772
(71) Applicant: Ube Industries, Ltd., Ube-shi, Yamaguchi-ken 755-8633 (JP)
(72) Inventor: NISHINO, S., c/o Ube Laboratories, Ube Ind. Ltd., Ube-shi, Yamaguchi 7558633 (JP); HIROTSU, K., c/o Ube Laboratories, Ube Ind. Ltd., Ube-shi, Yamaguchi 7558633 (JP); SHIMA, H., c/o Ube Laboratories, Ube Ind. Ltd., Ube-shi, Yamaguchi 7558633 (JP); SUZUKI, S., c/o Ube Laboratories, Ube Ind. Ltd., Ube-shi, Yamaguchi 7558633 (JP)
(74) Representative: Albrecht, Thomas
(86) International application number: PCT/JP2004/008640
(87) International publication number: WO 2004/113307

(57) **Abstract**

A pyrimidin-4-one compound can be prepared in a high yield by reacting an aminoarylcarboxylic acid compound with an organic acid compound in the presence of a nitrogen atom-containing compound under relatively simple and moderate reaction conditions.

## Description

### [Field of Invention]

The present invention relates to a method for preparing pyrimidin-4-one compounds.

### [Background of Invention]

The pyrimidin-4-one compounds such as quinazolin-4-one compounds, pyrazolopyrimidin-5-one compounds, and thienopyrimidinone compounds are useful compounds as starting compounds or intermediate compounds for preparing pharmaceutically active chemical compounds and agricultural chemical compounds.

Chem. Pharm. Bull., 46, 1926(1998) describes a method for preparing a pyrimidin-4-one by reacting anthranilic acid with formamide.

EP 1029853A describes a method for preparing 6-iodo-quinazolin-4-one by reacting 5-iodoanthranilic acid with formamidine acetate in ethanol for 20 hours.

J. Org. Chem., 18, 138(1953) describes a method for preparing quinazolin-4-one by reacting methyl anthranilate with formamide in the presence of ammonium formate.

J. Med. Chem., 41, 4021(1998) describes that 3-benzyl-2-butyl-3H-pyrido[3,2-d]pyrimidin-4-one hydrochloride is obtained with a yield of 8% by reacting 3-amino-2-pyridinecarboxylic acid with pentanoic acid anhydride at 140°C and further reacting the reaction product with benzylamine at 200°C.

WO 01/98284 describes that pyrazolopyrimidin-7-one is prepared with a yield of 7% by 4-amino-1-methyl-n-propyl-1H-pyrazol-5-carboxylate with a benzamidine compound in xylene.

Angew. Chem. Int. Ed. Engl., 7, 136(1968) describes a method of reacting an aminothiophenecarboxylic acid compound with a variety of nitrogen atom-containing compounds (e.g., formamide, nitriles, imino esters).

The above-mentioned various methods for preparing pyrimidin-4-one compounds have problems in the complicated reaction involved, yields, and the use of dangerous starting compounds.

### [Disclosure of Invention]

### [Object of Invention]

The present invention has a main object to provide a novel method for preparing a pyrimidin-4-one compound. Particularly, the invention has an object to provide a novel method for preparing a pyrimidin-4-one compound with a high yield under simple and moderate reaction conditions from easily available starting compounds having low dangerous properties.

### [Summary of Invention]

The present invention resides in a method for preparing a pyrimidin-4-one compound having the formula (5): 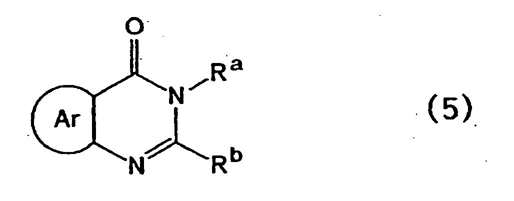 in which Ar represents an aromatic hydrocarbyl or heterocyclic ring optionally having a substituent, R^{a} represents hydrogen or a hydrocarbyl group, and R^{b} represents an atom or a group which does not participate in the below-mentioned reaction, provided that R^{b} is other than hydrogen where R^{a} is hydrogen;
which comprises reacting an aminoarylcarboxylic acid compound having the formula (1): 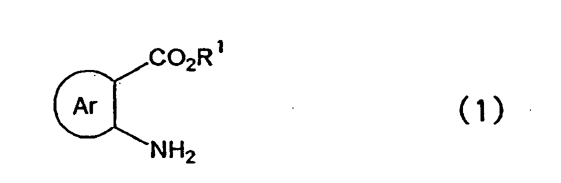 in which Ar has the above-mentioned meaning, and R¹ represents hydrogen or a hydrocarbyl group;
with an organic acid compound having the formula (4):

(R³O)₃CR^{b} (4)

in which R³ represents a hydrocarbyl group, and R^{b} has the above-mentioned meaning;
in the presence of a nitrogen atom-containing compound having the formula (2) or (3) :

R^{a}NH₂ (2)

R²CO₂NH₃R^{a} (3)

in which R² represents hydrogen or a hydrocarbyl group, and R^{a} has the above-mentioned meaning.

Preferred embodiments of the invention are described below.
(1) The reaction is performed in an organic solvent.
(2) The organic solvent is a polar solvent.
(3) The polar solvent is a lower alcohol having 1 to 6 carbon atoms.
(4) The nitrogen atom-containing compound is an amine compound or ammonium acetate.
(5) The reaction is performed at a temperature in the range of 40 to 200°C.
(6) Ar is a 5- or 6-membered aromatic hydrocarbyl ring optionally having a substituent.
(7) Ar is a 5- or 6-membered aromatic heterocyclic ring optionally having a substituent.
(8) The pyrimidin-4-one compound has the formula (7) : 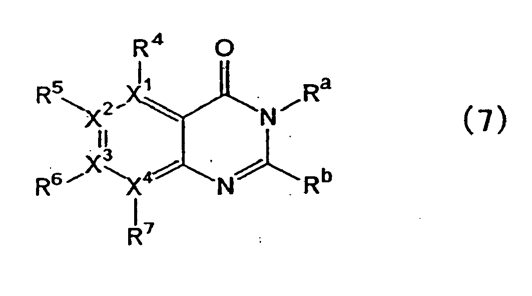 in which each of R^{a} and R^{b} has the meaning defined as above, each of R⁴, R⁵, R⁶ and R⁷ independently represents an atom or a group which does not participate in the reaction, provided that R⁴, R⁵, R⁶ and R⁷ can form a ring in optional combinations, and each of X¹, X², X³ and X⁴ independently represents a carbon atom or a nitrogen atom, provided that, where any of X¹, X², X³ and X⁴ are nitrogen atoms, the nitrogen atoms do not have the atom or group thereon,
   and the aminoarylcarboxylic acid compound is an aminocarboxylic acid compound having the formula (6): 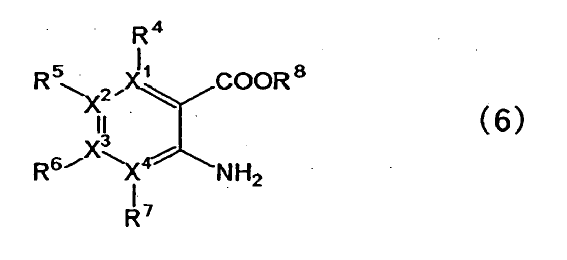 in which each of X¹, X², X³, X⁴, R⁴, R⁵, R⁶, and R⁷ has the meaning defined as above, and R⁸ represents an atom or a group which does not participate in the reaction.
(9) The pyrimidin-4-one compound is a quinazolin-4-one compound having the formula (9): 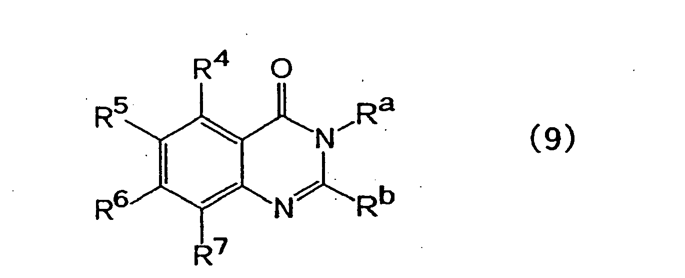 in which each of R^{a} and R^{b} has the meaning defined as above, each of R⁴, R⁵, R⁶ and R⁷ independently represents an atom or a group which does not participate in the reaction, provided that R⁴, R⁵, R⁶ and R⁷ can form a ring in optional combinations,
   and the aminoarylcarboxylic acid compound is an anthranilic acid having the formula (8): 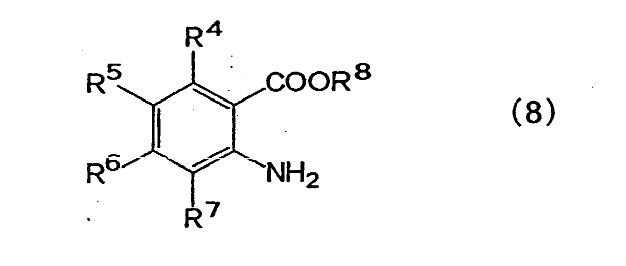 in which each of R⁴, R⁵, R⁶, and R⁷ has the meaning defined as above, and R⁸ represents an atom or a group which does not participate in the reaction.
(10) The pyrimidin-4-one compound is a pyrazolo-pyrimidin-7-one compound having the formula (11): 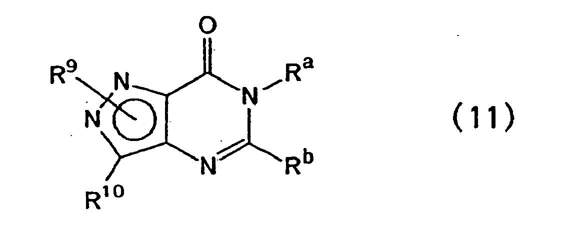 in which each of R^{a} and R^{b} has the meaning defined as above, each of R⁹ and R¹⁰ independently represents an atom or a group which does not participate in the reaction, provided that R⁹ and R¹⁰ can form a ring in combination,
   and the aminoarylcarboxylic acid compound is an aminopyrazolcarboxylic acid having the formula (10): 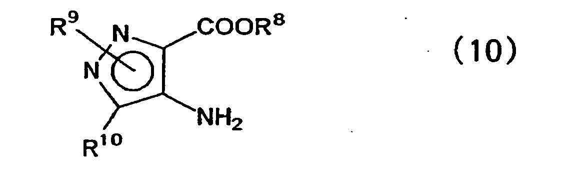 in which each of R⁹ and R¹⁰ has the meaning defined as above, and R⁸ represents an atom or a group which does not participate in the reaction.
(11) The pyrimidin-4-one compound is a thienopyrimidine compound having the formula (13): 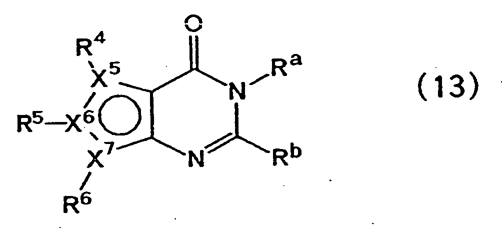 in which each of R^{a} and R^{b} has the meaning defined as above, each of R⁴, R⁵, and R⁶ independently represents an atom or a group which does not participate in the reaction, provided that R⁴, R⁵, and R⁶ can form a ring in optional combinations, and at least one of X⁵, X⁶ and X⁷ represents a sulfur atom, and other is carbon atom, provided that, where any of X⁵, X⁶ and X⁷ are sulfur atoms, the sulfur atoms do not have the atom or group thereon,
   and the aminoarylcarboxylic acid compound is an aminothiophenecarboxylic acid compound having the formula (12) : 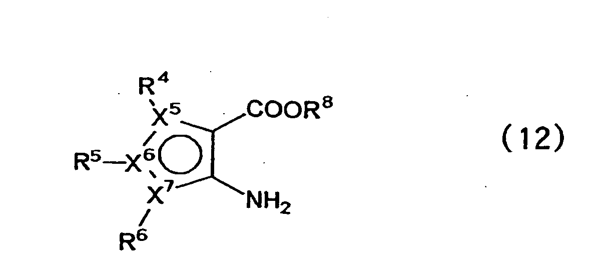
in which each of X⁴, X⁵, X⁶ , R⁴, R⁵, and R⁶ has the meaning defined as above, and R⁸ represents an atom or a group which does not participate in the reaction.

### [Effects of Invention]

The method of the invention enables to prepare pyrimidin-4-one compounds with high yields under simple and moderate reaction conditions from easily available starting compounds having low dangerous properties.

### [Preferred Embodiments of Invention]

Ar, R^{a}, R^{b}, R¹, R², R³, R⁴, R⁵, R⁶, R⁷, R⁸, R⁹, and R¹⁰ of the aforementioned formulas are described below in more detail.

Ar is an aromatic hydrocarbyl (or hydrocarbon) ring or an aromatic heterocyclic ring. These rings can have a substituent. Preferred are 5- or 6-membered aromatic hydrocarbyl rings and 5- or 6-membered aromatic heterocyclic rings which can have a substituent.

R^{a} is a hydrogen atom or a hydrocarbyl group. Examples of the hydrocarbyl group are alkyl groups having 1 to 12 carbon atoms such as methyl, ethyl, propyl, butyl, pentyl, hexyl, heptyl, octyl, nonyl, and decyl; cycloalkyl groups having 3 to 12 carbon atoms such as cyclopropyl, cyclobutyl, cyclopentyl, cyclohexyl, cycloheptyl, and cyclooctyl; aralkyl groups having 7 to 22 carbon atoms such as benzyl, phenethyl, and phenylpropyl; and aryl groups such as phenyl, p-tolyl, naphthyl, and anthryl.

R^{b} is an atom or a group which does not participate the reaction involved in the method of the invention. Examples include hydrogen atom, alkyl groups, cycloalkyl groups, aralkyl groups, aryl groups, halogen atoms, hydroxyl group, alkoxy groups, alkylthio groups, nitro group, cyano group, carbonyl group, amino groups, and carboxyl group. These groups can further have a substituent which does not participate in the reaction involved. Examples of the substituents are those described hereinbefore. Examples of the halogen atoms include fluorine, chlorine, bromine, and iodine. The alkylthio groups can be methylthio, ethylthio, or propylthio.

R¹ is a hydrogen atom or a hydrocarbyl (hydrocarbon) group. Examples of the hydrocarbyl groups are those described for R^{a}.

R² is a hydrogen atom or a hydrocarbyl (hydrocarbon) group. Examples of the hydrocarbyl groups are those described for R^{a}.

R³ is a hydrocarbyl (hydrocarbon) group. Examples of the hydrocarbyl groups are those described for R^{a}.

R⁴, R⁵, R⁶, R⁷, R⁸, R⁹ and R¹⁰ are the same as or different from each other, can have a substituent, and are groups which do not participate in the reaction. Examples of these groups include hydrogen atom, alkyl groups, cycloalkyl groups, aralkyl groups, aryl groups, halogen atoms, hydroxyl group, alkoxy groups, alkylthio groups, nitro group, cyano group, carbonyl group, amino groups (except for R⁴), and carboxyl group (except for R⁷).

The alkyl groups can be methyl, ethyl, propyl, butyl, pentyl, hexyl, heptyl, octyl, nonyl, or decyl. These groups can be in any isomer forms.

The cycloalkyl groups can be cyclopropyl, cyclobutyl, cyclopentyl, cyclohexyl, cycloheptyl, or cyclooctyl.

The aralkyl groups can be benzyl, phenethyl, or phenylpropyl. These groups can be any isomer forms.

The aryl groups can be phenyl, p-tolyl, naphthyl, or anthryl. These groups can be in any isomer forms.

The halogen atoms can be fluorine, chlorine, bromine, or iodine.

The alkoxy groups can be methoxy, ethoxy, or propoxy. These groups can be in any isomer forms.

The alkylthio groups can be methylthio, ethylthio, or propylthio. These groups can be in any isomer forms.

The alkyl groups, cycloalkyl groups, aralkyl groups, aryl groups, alkoxy groups, alkylthio groups and amino groups can have a substituent. The substituent can be a substituent connected via a carbon atom, a substituent connected via an oxygen atom, a substituent connected via a nitrogen atom, a substituent connected via a sulfur atom, or a halogen atom.

Examples of the substituents connected via a carbon atom include alkyl groups such as methyl, ethyl, propyl, butyl, pentyl, and hexyl, cycloalkyl groups such as cyclopropyl, cyclobutyl, cyclopentyl, and cyclohexyl, alkenyl groups such as vinyl, allyl, propenyl, cyclopropenyl, cyclobutenyl, and cyclopentenyl, heterocyclic groups such as pyrrolidyl, pyrrolyl, furyl, and thienyl, aryl groups such as phenyl, tolyl, xylyl, biphenylyl, naphthyl, anthryl, and phenanthryl, acyl groups (which can be acetallized) such as formyl, acetyl, propionyl, acryloyl, pivaloyl, cyclohexylcarbonyl, benzoyl, naphthoyl, and toluoyl, carboxyl groups, alkoxycarbonyl groups such as methoxycarbonyl and ethoxycarbonyl, aryloxycarbonyl groups such as phenoxycarbonyl, halogenated alkyl groups such as trifluoromethyl, and cyano group. These groups can be in any isomer forms.

Examples of the substituents connected via an oxygen atom include hydroxyl group, alkoxy groups such as methoxy, ethoxy, propoxy, butoxy, pentyloxy, hexyloxy, heptyloxy, benzyloxy, piperidyloxy, and pyranyloxy, and aryloxy groups such as phenoxy, tolyloxy, and naphthyloxy. These groups can be in any isomer forms.

Examples of the substituents connected via a nitrogen atom include primary amino groups such as methylamino, ethylamino, butylamino, cyclohexylamino, phenylamino, and naphthylamino, secondary amino groups such as dimethylamino, diethylamino, dibutylamino, methylethylamino, methybutylamino, and diphenylamino, heterocyclic amino groups such as morpholino, thiomorpholino, piperidino, piperazinyl, pyrazolidinyl, pyrrolidino, and indolyl. These groups can be in any isomer forms.

Examples of the substituents connected via a sulfur atom include mercapto group, thioalkoxy groups such as thiomethoxy, thioethoxy, and thiopropoxy, and thioaryloxy groups such as thiophenoxy, thiotolyloxy and thionaphthyloxy. These groups can be in any isomer forms.

Examples of the halogen atoms include fluorine, chlorine, bromine, and iodine.

The reaction involved in the preparation method of the invention is further described below.

The nitrogen atom-containing compound of the aforementioned formula (2) or (3) cam be employed preferably in an amount of 1 to 100 moles, more preferably in an amount of 3 to 40 moles, per one mole of the aminoarylcarboxylic acid. The nitrogen atom-containing compound can be in any of gas, liquid, and solid. Otherwise, the compound can be employed in a solution in an organic solvent such as a polar solvent (e.g., alcohol).

The organic acid compound of the formula (4) can be employed preferably in an amount of 1.0 to 15 moles, more preferably in an amount of 1.1 to 5.0 moles, per one mole of the aminoarylcarboxylic acid.

The reaction involved in the method of the invention can be carried out in the presence or absence of a solvent. There are no specific limitations with respect to the solvents, under the condition that the solvent does not give adverse effect to the reaction. Examples of the solvents include alcohols such as methanol, ethanol, isopropyl alcohol, n-butyl alcohol, t-butyl alcohol, and n-pentanol, amides such as N,N-dimethylformamide and N-methylpyrrolidone, ureas such as N,N'-dimethylimidazolidinone, sulfoxides such as dimethyl sulfoxide, aromatic hydrocarbons such as benzene, toluene, xylene, and mesitylene, halogenated hydrocarbons such as methylene chloride, chloroform, and dichloroethane, nitriles such as acetonitrile and propionitrile, and ethers such as diethyl ether, tetrahydrofuran, and dioxane. Preferred are alcohols, amides, and nitriles. More preferred are methanol, ethanol, N,N'-dimethylimidazolidinone, and acetonitrile. The solvents can be used singly or in combination.

The solvent can be employed preferably in an amount of 0 to 50 g, more preferably 0 to 20 g, most preferably 0 to 5 g, per one mole of the aminoarylcarboxylic acid. The amount may vary depending on the condition of the liquid reaction mixture and/or easiness for stirring.

The reaction involved in the method of the invention can be carried out by mixing and stirring the nitrogen atom-containing compound, aminoarylcarboxylic acid compound, organic acid compound, and solvent under inert gas atmosphere. The temperature for the reaction is preferably in the range of 40 to 200°C, more preferably 50 to 150°C. There is no limitation on pressure for the reaction.

The final product, i.e., the pyrimidin-4-one compound, can be isolated and purified after completion of the reaction by known methods such as extraction, filtration, concentration distillation, recrystallization, and/or column chromatography.

The invention is further described by the following examples.

### [Example 1] Synthesis of 6-iodo-2-methylquinazolin-4-one

In a pressure resistant, 10 mL-volume stainless steel vessel, 1.00 g (3.8 mmol) of 5-iodoanthranilic acid, 2.47 g (15.2 mmol) of ethyl orthoacetate, and 5.0 mL (38 mmol) of 15 wt.% ammonia-methanol solution were heated at 125°C for 8 hours for performing a reaction. After the reaction was complete, the reaction mixture was cooled to room temperature and concentrated. To the concentrated reaction mixture was added 20 mL of water, to precipitate a crystalline product. The crystalline product was collected by filtration, to give 0.94 g (yield after isolation: 86%) of 6-iodo-2-methylquinazolin-4-one as a white crystalline product.

The 6-iodo-2-methylquinazolin-4-one had the following properties:
¹H-NMR (DMSO-d₆, δ (ppm)) : 2.33 (3H, s), 7.36 (1H, d, J=8.5 Hz), 8.04 (1H, dd, J=8.6, 2.1 Hz), 8.35 (1H, d, J=2.0 Hz), 12.23 (1H, brs)
CI-MS (m/e) : 287 (M+1)

### [Example 2] Synthesis of 6-iodo-3-methylquinazolin-4-one

The procedures of Example 1 were repeated except that 2.47 g (15.2 mmol) of ethyl orthoacetate and 5.0 mL (38 mmol) of 15 wt.% ammonia-methanol solution were replaced with 1.61 g (15.2 mmol) of methyl orthoformate and 5.0 mL (28 mmol) of 20 wt.% methylamine-methanol solution, respectively. There was obtained 0.98 g (yield after isolation: 90%) of 6-iodo-3-methylquinazolin-4-one as a brownish gray crystalline product.

The 6-iodo-3-methylquinazolin-4-one had the following properties:
¹H-NMR (DMSO-d₆, δ(ppm)): 3.94 (3H, s), 7.46 (1H, d, J=8.4 Hz), 8.09 (1H, dd, J=8.4, 1.8 Hz), 8.40-8.42 (2H, m)
CI-MS (m/e): 287 (M+1)

### [Example 3] Synthesis of 6-iodo-2,3-dimethylquinazolin-4-one

The procedures of Example 1 were repeated except that 5.0 mL (38 mmol) of 15 wt.% ammonia-methanol solution were replaced with 5.0 mL (28 mmol) of 20 wt.% methylamine-methanol solution. There was obtained 0.83 g (yield after isolation: 73%) of 6-iodo-2,3-dimethyl-quinazolin-4-one as a white crystalline product.

The 6-iodo-2,3-dimethylquinazolin-4-one had the following properties:
¹H-NMR (DMSO-d₆, δ(ppm)): 2.56 (3H, s), 3.31 (3H, s), 3.52 (3H, s), 7.36 (1H, d, J=8.4 Hz), 8.04 (1H, dd, J=8.5, 1.8 Hz), 8.36 (1H, d, J=2.1 Hz)
CI-MS (m/e): 301 (M+1)

### [Example 4] Synthesis of 6-iodo-3-phenylquinazolin-4-one

In a pressure resistant, 20 mL-volume stainless steel vessel, 1.00 g (3.8 mmol) of 5-iodoanthranilic acid, 1.13 g (15.2 mmol) of ethyl orthoformate, 0.71 g (7.6 mmol) of aniline, and 10 mL of n-pentanol were heated at 125°C for 8 hours for performing a reaction. After the reaction was complete, the reaction mixture was cooled to room temperature and concentrated. To the concentrated reaction mixture was added 20 mL of water, to precipitate a crystalline product. The crystalline product was collected by filtration, to give 0.87 g (yield after isolation: 66%) of 6-iodo-3-phenylquinazolin-4-one as a white crystalline product.

The 6-iodo-3-phenylquinazolin-4-one had the following properties:
¹H-NMR (DMSO-d₆, δ(ppm)): 7.51-7.58 (6H, m), 8.17 (1H, dd, J=8.7, 2.4 Hz), 8.39 (1H, s), 8.46 (1H, d, J=2.1 Hz)
CI-MS (m/e) : 349 (M+1)

### [Example 5] Synthesis of 6-iodo-3-benzylquinazolin-4-one

In a pressure resistant, 20 mL-volume stainless steel vessel, 1.00 g (3.8 mmol) of 5-iodoanthranilic acid, 1.13 g (15.2 mmol) of ethyl orthoformate, 0.81 g (7.6 mmol) of benzylamine, and 10 mL of n-pentanol were heated at 125°C for 8 hours for performing a reaction. After the reaction was complete, the reaction mixture was cooled to room temperature and concentrated. To the concentrated reaction mixture was added 4 mL of 1 mol/L hydrochloric acid, to precipitate a crystalline product. The crystalline product was collected by filtration, to give 1.36 g (yield after isolation: 99%) of 6-iodo-3-benzylquinazolin-4-one as a white crystalline product.

The 6-iodo-3-benzylquinazolin-4-one had the following properties:
¹H-NMR (DMSO-d₆, δ(ppm)): 3.32 (2H, s), 7.32-7.36 (5H, m), 7.49 (1H, d, J=8.7 Hz), 8.11 (1H, d, J=2.1 Hz), 8.42 (1H, d, J=1.8 Hz), 8.61 (1H, s)
CI-MS (m/e): 363 (M+1)

### [Example 6] Synthesis of 3H-pyrido [2, 3-d]pyrimidin-4-one

In a pressure resistant, 10 mL-volume stainless steel vessel, 1.00 g (7.2 mmol) of 2-aminonicotinic acid, 3.07 g (28.8 mmol) of methyl orthoformate, and 5.0 mL (38 mmol) of 15 wt.% ammonia-methanol solution were heated at 105°C for 8 hours for performing a reaction. After the reaction was complete, the reaction mixture was cooled to room temperature and concentrated under reduced pressure, to give 1.06 g (yield after isolation: 100%) of 3H-pyrido[2,3-d]pyrimidin-4-one as a black solid product.

The 3H-pyrido[2,3-d]pyrimidin-4-one had the following properties:
¹H-NMR (DMSO-d₆, δ(ppm)): 3.36 (1H, brs), 7.46 (1H, dd, J=8.0, 4.5 Hz), 8.31 (1H, s), 8.45 (1H, dd, J=7.8, 2.1 Hz), 8.87 (1H, dd, J=4.8, 2.1 Hz)
CI-MS (m/e): 148 (M+1)

### [Reference Example 1] Synthesis of 4-amino-1-methyl-3-n-propyl-2H-pyrazole-5-carboxylic acid

In a 200 mL-volume glass reaction vessel equipped with a stirrer, a thermometer and a reflux condenser, 10.0 g (46.9 mmol) of 1-methyl-4-nitro-3-n-propyl-2H-pyrazol-5-carboxylic acid, 2 g of 5 wt.% Pd/C (water content: 50%), and 100 mL of ethanol were stirred at 50°C for 5 hours in a hydrogen atmosphere, for performing a reaction. After the reaction was complete, the reaction mixture was cooled to room temperature, filtered, and concentrated under reduced pressure, to give 8.0 g (yield after isolation: 80%) of 4-amino-1-methyl-3-n-propyl-2H-pyrazol-5-carboxylic acid as a red solid product.

The 4-amino-1-methyl-3-n-propyl-2H-pyrazol-5-carboxylic acid had the following properties:
¹H-NMR (DMSO-d₆, δ(ppm)): 0.98 (3H, t, J=7.6 Hz), 1.63-1.70 (2H, m), 2.51 (2H, t, J=7.2 Hz), 4.01 (3H, s), 6.96 (3H, brs)
CI-MS (m/e): 184 (M+1)

### [Example 7] Synthesis of 1-methyl-3-n-propyl-1,6-dihydro-pyrazolo[4,3-d]pyrimidin-7-one

In a pressure resistant, 10 mL-volume stainless steel vessel, 0.85 g (4.64 mmol) of 4-amino-1-methyl-3-n-propyl-2H-pyrazole-5-carboxylic acid prepared in Reference Example 1, 1.74 g (16.4 mmol) of methyl orthoformate, and 5.0 mL (38 mmol) of 15 wt.% ammonia-methanol solution were heated at 120°C for 8 hours for performing a reaction. After the reaction was complete, the reaction mixture was cooled to room temperature and concentrated under reduced pressure, to give 0.48 g (yield after isolation: 54%) of 1-methyl-3-n-propyl-1,6-dihydropyrazolo-[4,3-d]pyrimidin-7-one as a black solid product.

The 1-methyl-3-n-propyl-1,6-dihydropyrazolo[4,3-d]-pyrimidin-7-one had the following properties:
¹H-NMR (DMSO-d₆, δ (ppm)) : 0.91 (3H, t, J=7.5 Hz), 1.68-1.75 (2H, m), 2.74 (2H, t, J=7.2 Hz), 4.12 (3H, s), 7.80 (1H, s)
CI-MS (m/e): 193 (M+1)

### [Example 8] Synthesis of 3H-thieno[3,2-d]pyrimidin-4-one

In a pressure resistant, 10 mL-volume stainless steel vessel, 1.00 g (6.36 mmol) of methyl 3-aminothiophene-2-carboxylate, 2.02 g (19.1 mmol) of methyl orthoformate, and 5.0 mL (38 mmol) of 15 wt.% ammonia-methanol solution were heated at 130°C for 7 hours for performing a reaction. After the reaction was complete, the reaction mixture was cooled to room temperature and concentrated under reduced pressure. To the concentrated reaction mixture were successively added 50 mL of ethyl acetate and 50 mL of water. The aqueous portion was separated from the organic portion. The aqueous portion was concentrated to dryness under reduced pressure, to give 0.84 g (yield after isolation: 87%) of 3H-thieno [3,2-d]-pyrimidin-4-one as a black solid product.

The 3H-thieno[3,2-d]pyrimidin-4-one had the following properties:
¹H-NMR (DMSO-d₆, δ(ppm)): 7.29 (1H, d, J=5.1 Hz), 7.99 (1H, d, J=5.5 Hz), 8.12 (1H, s)
CI-MS (m/e): 153 (M+1)

### [Example 9] Synthesis of 6-iodo-2-methylquinazolin-4-one

In a pressure resistant, 10 mL-volume stainless steel vessel, 1.22 g (15.8 mmol) of ammonium acetate, 1.00 g (3.8 mmol) of 5-iodoanthranilic acid, 2.54 g (15.7 mmol) of ethyl orthoacetate, and 5.0 mL of methanol were heated at 130°C for 16 hours for performing a reaction. After the reaction was complete, the reaction mixture was cooled to room temperature and concentrated. To the concentrated reaction mixture was added 30 mL of water, to precipitate a crystalline product. The crystalline product was collected by filtration, to give 0.348 g (yield after isolation: 32%) of 6-iodo-2-methylquinazolin-4-one as a white crystalline product.

The 6-iodo-2-methylquinazolin-4-one had the following properties:
¹H-NMR (DMSO-d₆ , δ(ppm)): 2.33 (3H, s), 7.36 (1H, d, J=8.5 Hz), 8.04 (1H, dd, J=8.6, 2.1 Hz), 8.35 (1H, d, J=2.0 Hz), 12.23 (1H, brs)
CI-MS (m/e): 287 (M+1)

### [Example 10] Synthesis of 3H-thieno[3,2-d]pyrimidin-4-one

In a pressure resistant, 10 mL-volume stainless steel vessel, 2.04 g (26 mmol) of ammonium acetate, 1.00 g (6.36 mmol) of methyl 3-aminothiophene-2-carboxylate, 2.76 g (26 mmol) of methyl orthoformate, and 5.0 mL of methanol were heated at 60-70°C for 6 hours for performing a reaction. After the reaction was complete, the reaction mixture was cooled to room temperature and analyzed (according to absolute quantitative analysis) by high performance liquid chromatography. There was produced 0.91 g (reaction yield: 94%) of 3H-thieno [3,2-d] pyrimidin-4-one. The reaction mixture was then concentrated under reduced pressure. To the concentrated reaction mixture was added 40 mL of water, to precipitate a solid product, to give 0.26 g (yield after isolation: 27%) of 3H-thieno[3,2-d]pyrimidin-4-one as a brown solid product.

The 3H-thieno[3,2-d]pyrimidin-4-one had the following properties:
¹H-NMR (DMSO-d₆, δ(ppm)): 7.40 (1H, d, J=5.4 Hz), 8.15 (1H, s), 8.18 (1H, d, J=5.4 Hz), 12.48 (1H, brs)
CI-MS (m/e): 153 (M+1)

### [Example 11] Synthesis of 2-methyl-3H-pyrido[2,3-d]-pyrimidin-4-one

In a pressure resistant, 10 mL-volume stainless steel vessel, 1.63 g (21.1 mmol) of ammonium acetate, 0.70 g (5.07 mmol) of 2-aminonicotinic acid, 3.39 g (20.9 mmol) of methyl orthoacetate, and 3.2 mL of methanol were heated at 130°C for 16 hours for performing a reaction. After the reaction was complete, the reaction mixture was cooled to room temperature and concentrated under reduced pressure. The concentrated reaction mixture was washed with 30 mL of chloroform, and purified by silica gel chromatography (column: Wakogel C-200, developing solvent: ethyl acetate and methanol, in sequence) to give 0.40 g (yield after isolation: 49%) of 2-methyl-3H-pyrido[2,3-d]pyrimidin-4-one as a pale yellow solid product.

The 2-methyl-3H-pyrido[2,3-d]pyrimidin-4-one had the following properties:
¹H-NMR (DMSO-d₆, δ(ppm)): 2.33 (3H, s), 7.36 (1H, d, J=8.5 Hz), 8.04 (1H, dd, J=8.5, 2.2 Hz), 8.33 (1H, d, J=2.0 Hz), 12.33 (1H, brs)
CI-MS (m/e): 162 (M+1)

### [Example 12] Synthesis of 1,5-dihydropyrazolo[3,4-d]-pyrimidin-4-one

In a pressure resistant, 10 mL-volume stainless steel vessel, 1.99 g (25.8 mmol) of ammonium acetate, 1.00 g (7.09 mmol) of ethyl 5-amino-1H-pyrazole-4-carboxylate, 2.74 g (25.8 mmol) of methyl orthoformate, and 5.0 mL of methanol were heated at 130°C for 8 hours for performing a reaction. After the reaction was complete, the reaction mixture was cooled to room temperature and concentrated under reduced pressure, to give 0.85 g (yield after isolation: 88%) of 1,5-dihydropyrazolo[3,4-d]-pyrimidin-4-one as a black solid product.

The 1,5-dihydropyrazolo[3,4-d]-pyrimidin-4-one had the following properties:
¹H-NMR (DMSO-d₆, δ(ppm)): 3.35 (1H, brs), 8.01 (1H, s), 8.13 (1H, s), 12.83 (1H, brs)
CI-MS (m/e): 137 (M+1)

## Claims

1. A method for preparing a pyrimidin-4-one compound having the formula (5): 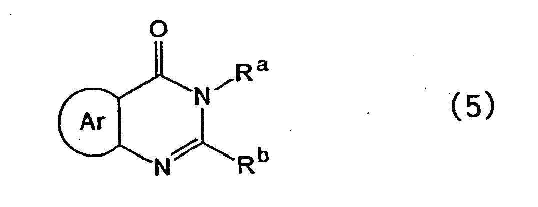 in which Ar represents an aromatic hydrocarbyl or heterocyclic ring optionally having a substituent, R^{a} represents hydrogen or a hydrocarbyl group, and R^{b} represents an atom or a group which does not participate in the below-mentioned reaction, provided that R^{b} is other than hydrogen where R^{a} is hydrogen;
which comprises reacting an aminoarylcarboxylic acid compound having the formula (1): 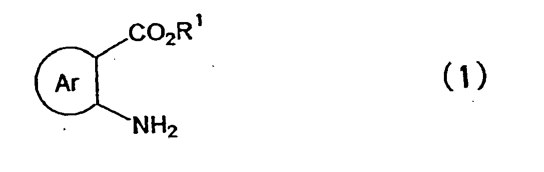 in which Ar has the above-mentioned meaning, and R¹ represents hydrogen or a hydrocarbyl group;
with an organic acid compound having the formula (4):
(R³O)₃CR^{b} (4)
in which R³ represents a hydrocarbyl group, and R^{b} has the above-mentioned meaning;
in the presence of a nitrogen atom-containing compound having the formula (2) or (3):
R^{a}NH₂ (2)
R²CO₂NH₃R^{a} (3)
in which R² represents hydrogen or a hydrocarbyl group, and R^{a} has the above-mentioned meaning.

2. The method of claim 1, in which the reaction is performed in an organic solvent.

3. The method of claim 2, in which the organic solvent is a polar solvent.

4. The method of claim 3, in which the polar solvent is a lower alcohol having 1 to 6 carbon atoms.

5. The method of claim 1, in which the nitrogen atom-containing compound is an amine compound or ammonium acetate.

6. The method of claim 1, in which the reaction is performed at a temperature in the range of 40 to 200°C.

7. The method of claim 1, in which Ar is a 5- or 6-membered aromatic hydrocarbyl ring optionally having a substituent.

8. The method of claim 1, in which Ar is a 5- or 6-membered aromatic heterocyclic ring optionally having a substituent.

9. The method of claim 1, in which the pyrimidin-4-one compound has the formula (7): 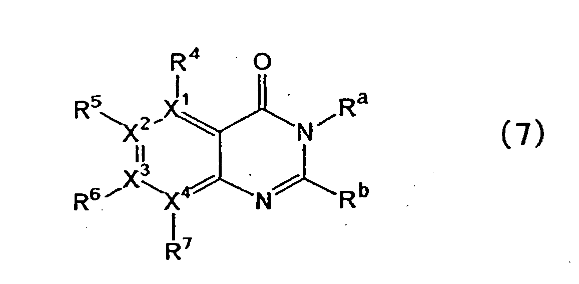 in which each of R^{a} and R^{b} has the meaning defined as above, each of R⁴, R⁵, R⁶ and R⁷ independently represents an atom or a group which does not participate in the reaction, provided that R⁴, R⁵, R⁶ and R⁷ can form a ring in optional combinations, and each of X¹, X², X³ and X⁴ independently represents a carbon atom or a nitrogen atom, provided that, where any of X¹, X², X³ and X⁴ are nitrogen atoms, the nitrogen atoms do not have the atom or group thereon,
and the aminoarylcarboxylic acid compound is an aminocarboxylic acid compound having the formula (6): 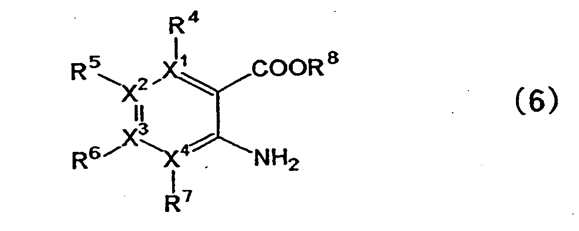 in which each of X¹, X², X³, X⁴, R⁴, R⁵, R⁶, and R⁷ has the meaning defined as above, and R⁸ represents an atom or a group which does not participate in the reaction.

10. The method of claim 1, in which the pyrimidin-4-one compound is a quinazolin-4-one compound having the formula (9): 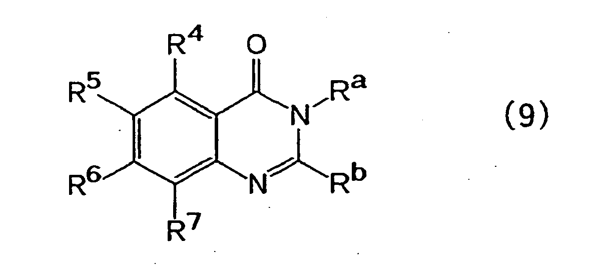 in which each of R^{a} and R^{b} has the meaning defined as above, each of R⁴, R⁵, R⁶ and R⁷ independently represents an atom or a group which does not participate in the reaction, provided that R⁴, R⁵, R⁶ and R⁷ can form a ring in optional combinations,
and the aminoarylcarboxylic acid compound is an anthranilic acid having the formula (8) : 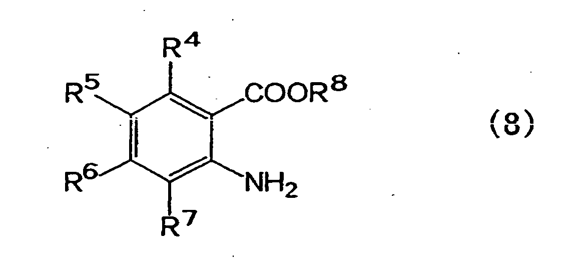 in which each of R⁴ , R⁵, R⁶ , and R⁷ has the meaning defined as above, and R⁸ represents an atom or a group which does not participate in the reaction.

11. The method of claim 1, in which the pyrimidin-4-one compound is a pyrazolopyrimidin-7-one compound having the formula (11): 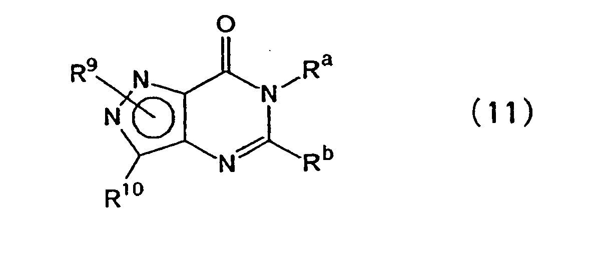 in which each of R^{a} and R^{b} has the meaning defined as above, each of R⁹ and R¹⁰ independently represents an atom or a group which does not participate in the reaction, provided that R⁹ and R¹⁰ can form a ring in combination,
and the aminoarylcarboxylic acid compound is an aminopyrazolcarboxylic acid having the formula (10): 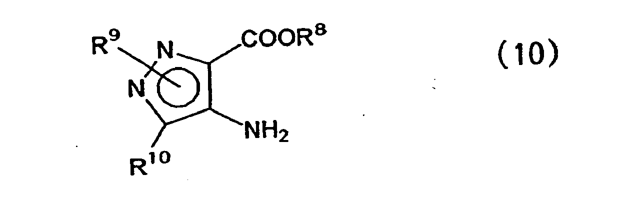 in which each of R⁹ and R¹⁰ has the meaning defined as above, and R⁸ represents an atom or a group which does not participate in the reaction.

12. The method of claim 1, in which the pyrimidin-4-one compound is a thienopyrimidine compound having the formula (13): 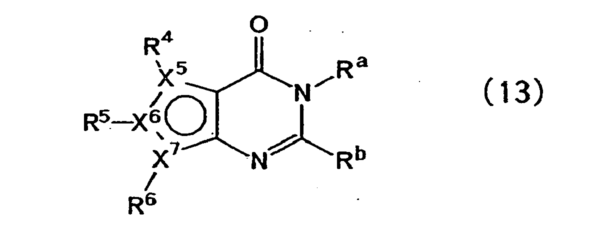 in which each of R^{a} and R^{b} has the meaning defined as above, each of R⁴, R⁵, and R⁶ independently represents an atom or a group which does not participate in the reaction, provided that R⁴, R⁵, and R⁶ can form a ring in optional combinations, and at least one of X⁵, X⁶ and X⁷ represents a sulfur atom, and other is carbon atom, provided that, where any of X⁵, X⁶ and X⁷ are sulfur atoms, the sulfur atoms do not have the atom or group thereon,
and the aminoarylcarboxylic acid compound is an aminothiophenecarboxylic acid compound having the formula (12) : 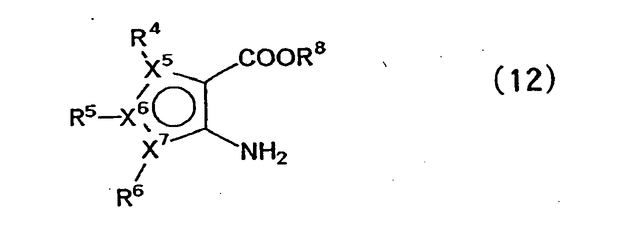 in which each of X⁴, X⁵, X⁶, R⁴, R⁵, and R⁶ has the meaning defined as above, and R⁸ represents an atom or a group which does not participate in the reaction.
